# EUROPEAN PATENT SPECIFICATION

(11) **EP 4 041 244 B1**
(45) Date of publication and mention of the grant of the patent: **15.07.2026**
(21) Application number: 20797912.1
(22) Date of filing: 05.10.2020
(51) Int. Cl.: A61K 31/53, A61P 1/04, A61P 35/02, A61P 43/00

(54) **AVAPRITINIB FOR USE IN THE TREATMENT OF EOSINOPHILIC DISORDERS**
AVAPRITINIB ZUR VERWENDUNG IN DER BEHANDLUNG VON EOSINOPHILSCHEN ERKRANKUNGEN
AVAPRITINIB POUR UTILISATION DANS LE TRAITEMENT DES MALADIES ÉOSINOPHILES

(30) Priority: 04.10.2019 US 201962910931 P
(43) Date of publication of application: 17.08.2022
(73) Proprietor: Blueprint Medicines Corporation, Cambridge, MA 02139 (US)
(72) Inventor: MAR, Brenton, Cambridge, MA 02139 (US)
(74) Representative: Mathys & Squire
(86) International application number: PCT/US2020/054221
(87) International publication number: WO 2021/067917

(56) References cited:
- WO-A1-2006/117185
- WO-A1-2013/059631
- CN-A- 109 970 745
- MICHAEL E. WECHSLER ET AL: "Novel targeted therapies for eosinophilic disorders", JOURNAL OF ALLERGY AND CLINICAL IMMUNOLOGY, vol. 130, no. 3, 1 September 2012 (2012-09-01), AMSTERDAM, NL, pages 563 - 571, XP055756420, ISSN: 0091-6749, DOI: 10.1016/j.jaci.2012.07.027
- VENKATESH PILLA REDDY ET AL: "The Pharmacokinetic-Pharmacodynamic (PKPD) Relationships of AZD3229, a Novel and Selective Inhibitor of KIT, in a Range of Mouse Xenograft Models of GIST", CLINICAL CANCER RESEARCH, vol. 26, no. 14, 15 July 2020 (2020-07-15), US, pages 3751 - 3759, XP055756234, ISSN: 1078-0432, DOI: 10.1158/1078-0432.CCR-19-2848
- JASON G. KETTLE ET AL: "Discovery of N -(4-{[5-Fluoro-7-(2-methoxyethoxy)quinazolin-4-yl]amino}phenyl)-2-[4-(propan-2-yl)-1 H -1,2,3-triazol-1-yl]acetamide (AZD3229), a Potent Pan-KIT Mutant Inhibitor for the Treatment of Gastrointestinal Stromal Tumors", JOURNAL OF MEDICINAL CHEMISTRY, vol. 61, no. 19, 11 September 2018 (2018-09-11), pages 8797 - 8810, XP055756283, ISSN: 0022-2623, DOI: 10.1021/acs.jmedchem.8b00938

## Description

### BACKGROUND

Eosinophils are bone marrow-derived granulocytes implicated beneficially in host defense against infection, in anti-tumor cytotoxicity, and in wound healing. Eosinophils are implicated in various diseases including allergic diseases and are thought to play an important role in generating morbidity of allergic diseases, such as chronic bronchial asthma and atopic dermatitis (Adv. Immunol., 39, 177 (1986), Immunol. Today, 13, 501 (1992)). In addition to the above diseases, eosinophils are also implicated in diseases generally referred to as hypereosinophilic syndrome (HES), eosinophilia, eosinophilic enterogastritis, eosinophilic leukemia, eosinophilic granuloma and Kimura's disease (Ann. Intern. Med., 97, 78 (1982)).

Eosinophils are divided into subgroups, normodense eosinophils and hypodense eosinophils. Eosinophils have been shown to be hypodense eosinophils upon activation (Immunology, 47, 531 (1982)). Hypodense eosinophils are also referred to as activated eosinophils. It has been reported that a qualitative change occurs in addition to a quantitative change in eosinophils in the peripheral blood of an HES patients (Clin. Exp. Immunol., 24, 423 (1976)). Activated eosinophils have been implicated in the severity of HES symptom (Am. J. Cardiol., 52, 321 (1983)). Aside from HES patients, activated eosinophils have been also found in the peripheral blood, and in bronchoalveolar lavage fluid (BALE) of patients with bronchial asthma (Am. Rev. Respir. Dis, 132, 981 (1985)). Various receptors, such as those of cytokines, are expressed on activated eosinophils (hypodense eosinophils) (J. Immunol., 142, 4416 (1989)). Compared to normodense eosinophils, these hypodense eosinophils show elevated sensitivities against IL-5 (Clin. Exp. Immunol., 85, 312 (1991); J. Exp. Med., 172, 1347 (1990)).

Currently, treatment for patients with eosinophilic diseases consists of administration of steroids. However, steroid administration is often associated with side effects. Specifically, the treatment has some other problems, such that patient's pathological condition may return to the original state when steroid administration is discontinued, and prolonged steroid administration may induce steroid resistance. Accordingly, there is a need for safe and effective treatments for eosinophil related disorders. J Allergy Clin. Immunol., 130, 563-571 (2012) discusses targeted therapies for eosinophilic disorders.

### SUMMARY

It now has been found that Compound (I) as shown below effectively reduces eosinophil levels (Example 1) in patients. Based on this discovery, a Compound (I) or a pharmaceutically acceptable salt thereof for use in reducing eosinophil levels in patients is disclosed herein.

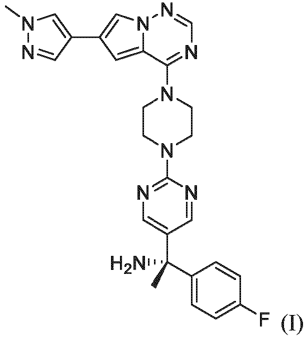

One aspect, the present invention provides Compound (I) or a pharmaceutically acceptable salt thereof for use in treating an eosinophilic disorder, as defined in the appended claims.

### DESCRIPTION OF THE DRAWINGS

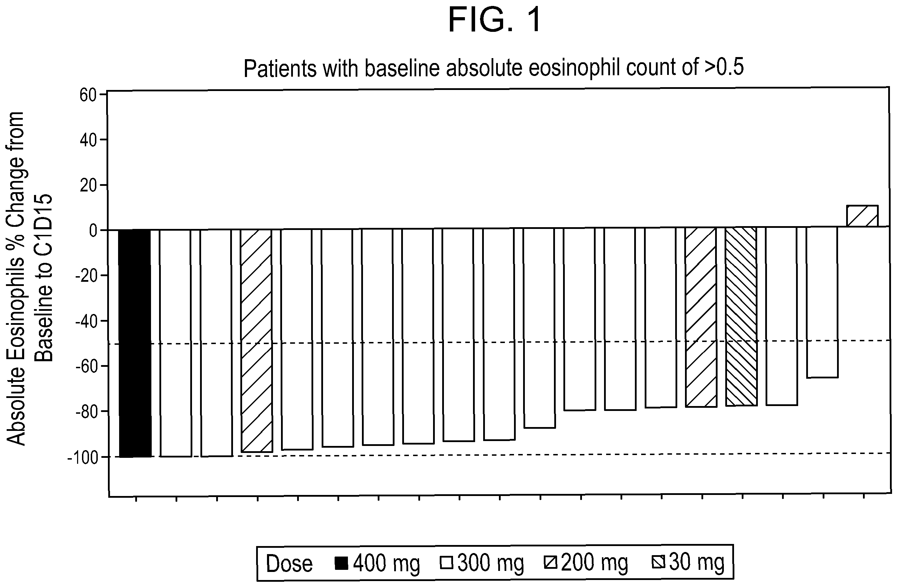

Figure 1 shows the absolute eosinophil changes from baseline levels for C1D15 (cycle 1 day 15) in adult patients treated with Compound (I) in a Phase 1 clinical trial after 2 weeks. The dose range of Compound (I) is between 30 mg and 400 mg. The patients with baseline absolute eosinophil count of >0.5.

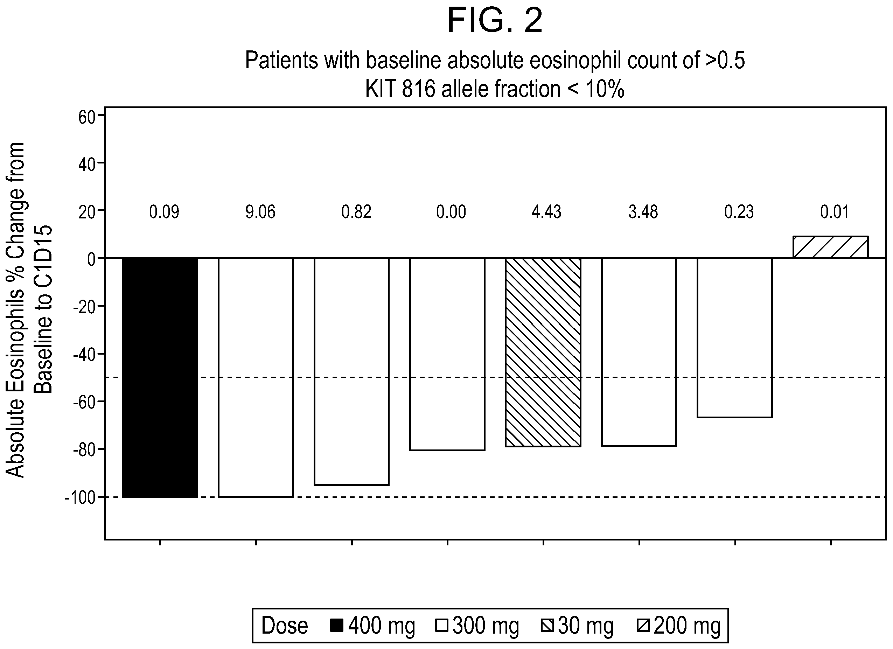

Figure 2 shows the absolute eosinophil changes from baseline levels for C1D15 (cycle 1 day 15) in adult patients with KIT allele fraction < 10% treated with Compound (I) in a Phase 1 clinical trial after 2 weeks. The dose range of Compound (I) is between 30 mg and 400 mg. The patients with baseline absolute eosinophil count of >0.5.

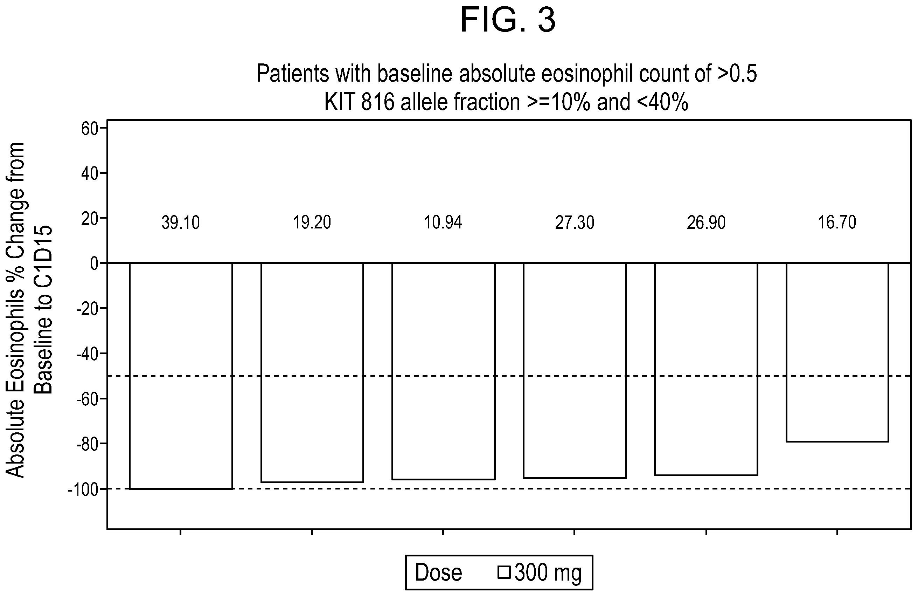

Figure 3 shows the absolute eosinophil changes from baseline levels for C1D15 (cycle 1 day 15) in adult patients with KIT allele fraction between 10% and 40% treated with Compound (I) (with a dose of 300 mg) in a Phase 1 clinical trial after 2 weeks. The patients with baseline absolute eosinophil count of >0.5.

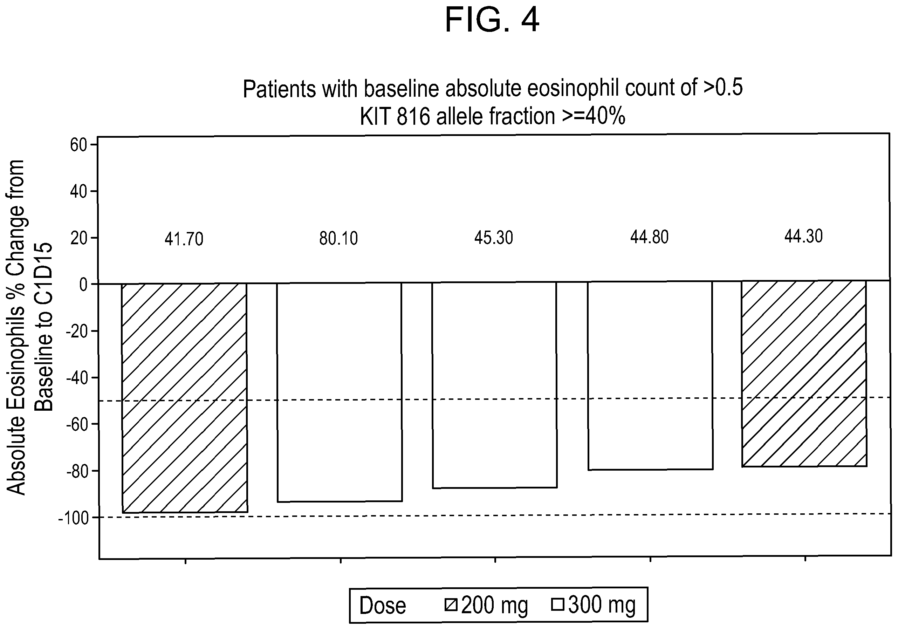

Figure 4 shows the absolute eosinophil changes from baseline levels for C1D15 (cycle 1 day 15) in adult patients with KIT allele fraction > 40% treated with Compound (I) in a Phase 1 clinical trial after 2 weeks. The dose range of Compound (I) is between 200 mg and 300 mg. The patients with baseline absolute eosinophil count of >0.5.

### DETAILED DESCRIPTION

Eosinophils have been implicated in the pathogenesis of numerous diseases and disorders. Eosinophils are a type of white blood cell, which help fight off infections and play a role in body's immune response. They are a normal cellular component of the blood and also of certain tissues, including spleen, lymph nodes, thymus, and the submucosal areas of the gastrointestinal, respiratory, and genitourinary tracts. Counts of 0 to 450 eosinophils per cubic millimeter of blood are considered within normal limits.

Eosinophilic disorders occur when eosinophils are found in above-normal amounts in various parts of the body and/or when there is a higher than normal ratio of hypodense versus normodense eosinophils (*e.g*., greater than 30%). The eosinophilic disorder described herein are characterized by an overabundance of eosinophils (eosinophilia). The increased number of eosinophils inflame tissues and cause organ damage. The heart, lungs, skin, and nervous system are most often affected, but any organ can be damaged.

Eosinophilic disorders are diagnosed according to the location where the levels of eosinophils are elevated:
Eosinophilic pneumonia (lungs)
Eosinophilic cardiomyopathy (heart)
Eosinophilic esophagitis (esophagus - EoE)
Eosinophilic gastritis (stomach - EG)
Eosinophilic gastroenteritis (stomach and small intestine - EGE)
Eosinophilic enteritis (small intestine)
Eosinophilic colitis (large intestine - EC)
Hypereosinophilic syndrome (blood and any organ - HES)

Additionally, non-limiting examples of diseases and disorders in which eosinophils play a role *(i.e.,* eosinophilic disorders) are: asthma, immunoglobulin (IgE)-mediated food allergy, eosinophilic esophagitis (inflammation of the esophagus), inflammatory bowel disease, COPD, allergic colitis, astro-esophageal reflux, eosinophilic gastrointestinal disease (EGID), eosinophilic gastroenteritis, endomyocardial fibrosis, Loeffler's endocarditis. Davies disease, episodic angioedema associated with eosinophilia, eosinophilia-myalgia syndrome/Spanish toxic oil syndrome, liver cirrhosis, dermatitis herpetiformis, Bullous pemphigoid, Churg-Strauss syndrome, acute myelogenous eosinophilic leukemia, acute lymphocytic eosinophilic leukemia, systemic mastocytosis with eosinophilia, allergic rhinitis, eczema, Wegener's granulomatosis, polyarteritis nodosa, eosinophilic fasciitis, and rheumatoid arthritis.

Accordingly, one aspect of the present invention is a Compound (I) or a pharmaceutically acceptable salt thereof for use in treating an eosinophilic disorder. In one embodiment, the eosinophilic disorder is selected from hypereosinophilic syndrome, eosinophilia, eosinophilic enterogastritis, eosinophilic leukemia, eosinophilic granuloma and Kimura's disease.

In one embodiment, the eosinophilic disorder is hypereosinophilic syndrome. In a specific embodiment, the hypereosinophilic syndrome is idiopathic hypereosinophilic syndrome. In one embodiment, the eosinophilic disorder is eosinophilic leukemia. In a specific embodiment, the eosinophilic leukemia is chronic eosinophilic leukemia. In another embodiment, the eosinophilic disorder is refractory to treatment with imatinib, sunitinib, and/or regorafenib. In a specific embodiment, the eosinophilic disorder is refractory to treatment with imatinib.

One aspect of the present disclosure, which is not expressly recited in the appended claims, is a method of reducing the number of eosinophils in a subject in need thereof comprising administering to the subject a therapeutically effective amount of Compound (I) or a pharmaceutically acceptable salt thereof.

References to methods of treatment throughout this description are to be interpreted as references to compounds and pharmaceutical compositions of the present disclosure for use in those methods of treatment.

In one embodiment, the disclosed methods reduce the number of eosinophils in the blood, bone marrow, gastrointestinal tract (*e.g*., esophagus, stomach, small intestine and colon), or lung. In another embodiment, a method disclosed herein reduces the number of blood eosinophils. In a further embodiment, a method disclosed herein reduces the number of lung eosinophils. In still a further embodiment, a method disclosed herein reduces the number of eosinophil precursor cells.

In another embodiment, the disclosed methods reduce (post-administration) the number of eosinophils by at least 10%, at least 20%, at least 30%, at least 40%, at least 50%, at least 60%, at least 70%, at least 80%; at least 90%, at least 95% or at least 99%. In a specific embodiment, a method disclosed herein reduces the number of eosinophils below the limit of detection.

In another embodiment, the disclosed methods reduce (post-administration) the number of eosinophil precursors by at least 10%, at least 20%, at least 30%, at least 40%, at least 50%, at least 60%, at least 70%, at least 80%, at least 90%, at least 95% or at least 99%. In a specific embodiment, a method disclosed herein reduces the number of eosinophil precursors below the limit of detection.

In a further embodiment, which is not expressly recited in the appended claims, the disclosed methods reduce eosinophils to a level below detection following a single administration of Compound (I) or a pharmaceutically acceptable salt thereof. In a specific embodiment, a single administration of Compound (I) or a pharmaceutically acceptable salt thereof reduces eosinophils to a level below detection for at least 1 day, at least 2 days, at least 3 days, at least 4 days, at least 5 days, at least 6 days, at least 7 days, at least 2 weeks, at least 3 weeks, at least 4 weeks, at least 5 weeks, at least 6 weeks, at least 7 weeks, at least 8 weeks, at least 9 weeks, at least 10 weeks, at least 12 weeks, at least 14 weeks, at least 16 weeks, at least 20 weeks, or at least 25 weeks.

In a further embodiment, which is not expressly recited in the appended claims, the disclosed methods reduce eosinophil precursors following a single administration of Compound (I) or a pharmaceutically acceptable salt thereof. In a specific embodiment, a single administration of Compound (I) or a pharmaceutically acceptable salt thereof reduces eosinophil precursors to a level below detection for at least 1 day, at least 2 days, at least 3 days, at least
4 days, at least 5 days, at least 6 days, at least 7 days, at least
2 weeks, at least 3 weeks, at least 4 weeks, at least 5 weeks, at least
6 weeks, at least 7 weeks, at least 8 weeks, at least 9 weeks, at least 10 weeks, at least 12 weeks, at least 14 weeks, at least 16 weeks, at least 20 weeks, or at least 25 weeks.

In another aspect of the disclosure, which is not expressly recited in the appended claims, there is provided a method of treating an eosinophilic disorder, comprising administering to a subject in need thereof an amount of 30 mg to 400 mg (*e.g.,* 100 mg to 300 mg, or 200 mg to 300 mg) of Compound (I) and/or a pharmaceutically acceptable salt thereof once a day. In some embodiments, the amount is 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg once a day. In some embodiments, the amount is 25 mg once a day. In some embodiments, the amount is 50 mg once a day. In some embodiments, the amount is 75 mg once a day. In some embodiments, the amount is 100 mg once a day. In some embodiments, the amount is 150 mg once a day. In some embodiments, the amount is 200 mg once a day. In some embodiments, the amount is 250 mg once a day. In some embodiments, the amount is 300 mg once a day.

As used herein, "Compound (I)" refers to a compound having a chemical name (S)-1-(4-fluorophenyl)-1-(2-(4-(6-(1-methyl-1H-pyrazol-4-y)pyrrolo[2,1-f][1,2,4]triazin-4-yl)piperazin-yl)pyrimidin-5-yl)ethan-1-amine, which has the following structure:

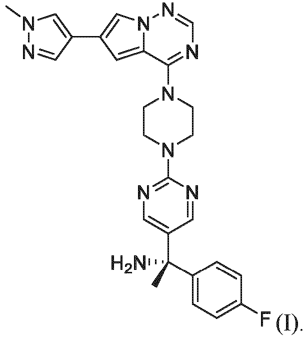

Compound (I) is disclosed in WO 2015/057873. The preparation of Compound (I) is described in Example 7 of WO 2015/057873.

Compound (I) was developed to selectively target KIT D816V and other KIT exon 17 mutations, and has demonstrated potent and selective activity against KIT D816V *in vitro,* robust growth inhibition in a tyrosine kinase inhibitor (TKI)-resistant mastocytoma model *in vivo,* and tolerability at active doses in toxicology and safety pharmacology studies. An ongoing Phase 1 study of Compound (I) in patients with advanced systemic mastocytosis (AdvSM) (NCT02561988) is evaluating safety and preliminary efficacy. The recommended Phase 2 dose (RP2D) was identified as 300 mg once a day (QD), and an expansion cohort of the study is further evaluating efficacy and safety of this dose in a larger cohort of patients, as well as validating the AdvSM Symptom Assessment Form (AdvSM-SAF) that has been developed to assess the impact of Compound (I) on symptom improvement in patients with AdvSM. Based on emerging safety and efficacy data in patients treated at 300 mg QD, an additional cohort of patients treated at 200 mg QD was added.

Activating mutations at the D816 position are found in eosinophilic disorders, with the most common mutations being D816V and D816Y. The D816V mutation is found in the activation loop of the kinase domain and leads to constitutive activation of KIT kinase.

Primary treatment with a KIT inhibitor such as imatinib has also been shown to be beneficial for initial treatment of eosinophilic disorders. Specifically, imatinib is approved for the treatment of idocratic hypereosinophilic syndrome. However, resistance to imatinib occurs within months through somatic mutation. These secondary imatinib resistant mutations are most frequently located on Exon 11, 13, 14, 17 or 18. There is a need for therapeutic agents to treat patients with eosinophilic disorders, specifically patients who have exon 17 mutations.

Compound (I) or a pharmaceutically acceptable salt thereof can be active against one or more KIT mutations in Exon 17 (*e.g.,* D816V, D816Y, D816F, D816K, D816H, D816A, D816G, D820A, D820E, D820G, N822K, N822H, Y823D, and A829P), and much less active against wild-type KIT.

In one embodiment, Compound (I) or a pharmaceutically acceptable salt thereof can be active against a D816 mutation in KIT in Exon 17. In a specific embodiment, the D816 mutation is D816V. In another specific embodiment, the D816 mutation is D816Y.

As used herein, the term "pharmaceutically acceptable salt" refers to a non-toxic salt form of a compound of this disclosure. Pharmaceutically acceptable salts of Compound (I) include those derived from suitable inorganic and organic acids and bases. Pharmaceutically acceptable salts are well known in the art. Suitable pharmaceutically acceptable salts are, *e.g.,* those disclosed in Berge, S.M., et al. J. Pharma. Sci. 66:1-19 (1977). Non-limiting examples of pharmaceutically acceptable salts disclosed in that article include: acetate; benzenesulfonate; benzoate; bicarbonate; bitartrate; bromide; calcium edetate; camsylate; carbonate; chloride; citrate; dihydrochloride; edetate; edisylate; estolate; esylate; fumarate; gluceptate; gluconate; glutamate; glycollylarsanilate; hexylresorcinate; hydrabamine; hydrobromide; hydrochloride; hydroxynaphthoate; iodide; isethionate; lactate; lactobionate; malate; maleate; mandelate; mesylate; methylbromide; methylnitrate; methylsulfate; mucate; napsylate; nitrate; pamoate (embonate); pantothenate; phosphate/diphosphate; polygalacturonate; salicylate; stearate; subacetate; succinate; sulfate; tannate; tartrate; teociate; triethiodide; benzathine; chloroprocaine; choline; diethanolamine; ethylenediamine; meglumine; procaine; aluminum; calcium; lithium; magnesium; potassium; sodium; and zinc.

Non-limiting examples of pharmaceutically acceptable salts derived from appropriate acids include: salts formed with inorganic acids, such as hydrochloric acid, hydrobromic acid, phosphoric acid, sulfuric acid, or perchloric acid; salts formed with organic acids, such as acetic acid, oxalic acid, maleic acid, tartaric acid, citric acid, succinic acid, or malonic acid; and salts formed by using other methods used in the art, such as ion exchange. Additional non-limiting examples of pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, and valerate salts. Non-limiting examples of pharmaceutically acceptable salts derived from appropriate bases include alkali metal, alkaline earth metal, ammonium, and N+(C1-4 alkyl)4 salts. This disclosure also envisions the quaternization of any basic nitrogen-containing groups of the compounds disclosed herein. Non-limiting examples of alkali and alkaline earth metal salts include sodium, lithium, potassium, calcium, and magnesium. Further non-limiting examples of pharmaceutically acceptable salts include ammonium, quaternary ammonium, and amine cations formed using counterions such as halide, hydroxide, carboxylate, sulfate, phosphate, nitrate, lower alkyl sulfonate, and aryl sulfonate. Other non-limiting examples of pharmaceutically acceptable salts include besylate and glucosamine salts.

As used herein, "a therapeutically effective amount" of a compound disclosed herein refers to an amount of the compound that will elicit a biological or medical response in a subject, *e.g.*, reduce or inhibit enzyme or protein activity, ameliorate symptoms, alleviate conditions, or slow or delay disease progression.

As used herein, the term "patient" or "subject" refers to an organism to be treated by the methods of the disclosure. Non-limiting example organisms include mammals, *e.g.*, murines, simians, equines, bovines, porcines, canines, felines, and the like. In some embodiments, the organism is a human.

As used herein, the term "treat," "treating," or "treatment," when used in connection with a disorder or condition, includes any effect, *e.g.*, lessening, reducing, modulating, ameliorating, and/or eliminating, that results in the improvement of the disorder or condition. Improvements in or lessening the severity of any symptom of the disorder or condition can be readily assessed according to standard methods and techniques known in the art.

Hypereosinophilic syndrome is a group of blood disorders that are characterized by high numbers of eosinophils - white blood cells that play an important role in immune system. Over time, the excess eosinophils enter various tissues, eventually damaging organs.

The contemporary classification of HES includes (1) idiopathic HES in which the cause of HE remains unknown, (2) primary (neoplastic) HES in which eosinophils are found to be clonal, and (3) secondary (reactive) HES with the presence of cytokine-driven nonclonal HE. A unique form of secondary HES is called lymphocytic in which aberrant T-cells can be identified in blood.

### Pharmaceutical Compositions

Compound (I) and/or pharmaceutically acceptable salts thereof described herein are useful as an active pharmaceutical ingredients (API) as well as materials for preparing pharmaceutical compositions that incorporate one or more pharmaceutically acceptable excipients and is suitable for administration to human subjects.

In some embodiments, the disclosure provides a pharmaceutical composition comprising Compound (I) and/or a pharmaceutically acceptable salt thereof and at least one additional pharmaceutically acceptable excipient. The term "pharmaceutically acceptable excipient," as used herein, refers to a pharmaceutically acceptable material, composition, and/or vehicle, such as a liquid or solid filler, diluent, excipient, solvent, or encapsulating material. Each excipient must be "pharmaceutically acceptable" in the sense of being compatible with the subject composition and its components and not injurious to the patient. Except insofar as any conventional pharmaceutically acceptable excipient is incompatible with Compound (I) and/or pharmaceutically acceptable salts thereof, such as by producing any undesirable biological effect or otherwise interacting in a deleterious manner with any other component(s) of the pharmaceutically acceptable composition, its use is contemplated to be within the scope of this disclosure.

Some non-limiting examples of materials which may serve as pharmaceutically acceptable excipients include: (1) sugars, such as lactose, glucose, and sucrose; (2) starches, such as com starch and potato starch; (3) cellulose and its derivatives, such as sodium carboxymethyl cellulose, ethyl cellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) glycols, such as propylene glycol; (11) polyols, such as glycerin, sorbitol, mannitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York also disclose additional non-limiting examples of pharmaceutically acceptable excipients, as well as known techniques for preparing and using the same.

Pharmaceutical compositions disclosed herein may be administered orally, parenterally, by inhalation spray, topically, rectally, nasally, buccally, vaginally, or via an implanted reservoir. The term "parenteral," as used herein includes subcutaneous, intravenous, intramuscular, intra-articular, intra-synovial, intrasternal, intrathecal, intrahepatic, intralesional, and intracranial injection or infusion techniques. In some embodiments, the compositions of the disclosure are administered orally, intraperitoneally, or intravenously. Sterile injectable forms of the pharmaceutical compositions of this disclosure may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example, as a solution in 1,3-butanediol. Non-limiting examples of acceptable vehicles and solvents that may be employed are water, Ringer's solution, and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives, are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tween, Spans, and other emulsifying agents or bioavailability enhancers which are commonly used in the manufacture of pharmaceutically acceptable solid, liquid, or other dosage forms may also be used for the purposes of formulation.

Pharmaceutical compositions disclosed herein may also be orally administered in any orally acceptable dosage form including, but not limited to, capsules, tablets, aqueous suspensions, or solutions. When aqueous suspensions are required for oral use, the active ingredient is typically combined with emulsifying and suspending agents. If desired, certain sweetening, flavoring, or coloring agents may also be added. In some embodiments, the pharmaceutical composition comprising Compound (I) and/or a pharmaceutically acceptable salt thereof is a tablet prepared using methods known in the art. In some embodiments, the tablet is an immediate release tablet for oral administration. In some embodiments, Compound (I) and/or a pharmaceutically acceptable salt thereof is blended with pharmacopeial excipients to form an immediate release tablet. In some embodiments, the excipients comprising the tablet are microcrystalline cellulose, copovidone, croscarmellose sodium, and magnesium stearate. In some embodiments, the formulation blend is roller compacted, compressed into round tablets, and aesthetically film coated.

### EXEMPLIFICATION

The following examples are intended to be illustrative.

### Example 1

This is a Phase 1, open-label, study designed to evaluate the safety, tolerability, PK, PD, and preliminary antineoplastic activity of Compound (I), administered orally, in adult patients with advanced systemic mastocytosis (AdvSM) and relapsed or refractory myeloid malignancies.

The study consists of 2 parts: dose-escalation (Part 1) and expansion (Part 2). Approximately 25 patients were in Part 1 and approximately 55 patients were in Part 2.

Systemic mastocytosis diagnosis confirmation for study eligibility is to be assessed using the WHO criteria for diagnosis and subclassification. Evaluable C-findings per modified IWG-MRT-ECNM criteria are to be used to assess response.

A treatment cycle is 28 days in duration. Patients presented to the study center on C1D1 for the first dose of study drug and serial PK sampling, PD sample collection, vital sign measurement, electrocardiogram (ECG) monitoring, patient general impression of symptom severity (PGIS) and European Organization for Research and Treatment of Cancer Core Quality of Life Questionnaire (EORTC QLQ-C30), safety monitoring and adverse event (AE) recording.

Advanced SM response were assessed by imaging (magnetic resonance imaging [MRI] or computed tomography [CT]) and bone marrow (BM) evaluation at C3D1, C5D1 (imaging only), C7D1, C11D1, and C18D1. After C18, AdvSM response were assessed every 6 cycles.

All patients attended an end of treatment (EOT) visit within 14 (± 7) days after the last dose of study drug. The EOT visit must occur no more than 21 days after last dose of study drug.

### Part 1 (Dose Escalation)

Patients with AdvSM or a relapsed or refractory myeloid malignancy were enrolled in the dose escalation part of the study. A 3+3 dose-escalation design using cohorts of 3 patients were employed.

The first cohort of patients received Compound (I) at a starting dose of 30 mg once daily (QD). Dose escalation proceeded at increments up to 100% until ≥ 1 patient treated at a given dose level has a ≥ Grade 2 non-hematologic AE or a Grade 4 hematologic AE and the AE (non-hematologic or hematologic) was not clearly attributable to a cause other than Compound (I) or the dose exceeds the highest dose determined to be safe in the ongoing Compound (I) first-in-human (FIH) study in GIST, BLU-285-1101.

Three patients were enrolled initially in each cohort and an additional 3 patients (for a total of 6) were enrolled when the cohort requires expansion due to dose-limiting toxicity (DLT). Dose escalation continued until the maximum tolerated dose (MTD) or a recommended phase 2 dose (RP2D) below the MTD were determined.

### Part 2 (Expansion)

Once the MTD or RP2D were determined, up to 3 groups of patients with the following diagnoses of AdvSM were enrolled in Part 2 and treated with Compound (I) at the RP2D.
- Patients with ASM.
- Patients with SM-AHN.
- Patients with MCL.

Based on available efficacy, PK, and long-term safety data, Compound (I) 200 mg QD were selected as the starting dose for the remainder of the study; all patients newly enrolled into Part 2 of the study received 200 mg as their starting dose.

Part 2 enrolled 2 cohorts. Patients who do not have measurable C-findings per modified IWG-MRT-ECNM criteria at Baseline, attributed to SM, were enrolled in cohort 1. Patients who have at least 1 measurable C-finding per modified IWG-MRT-ECNM criteria at Baseline, attributed to SM, were enrolled in cohort 2. The patients in Cohort 2 was the evaluable population for the primary objective of determining ORR, based on modified IWG-MRT-ECNM as adjudicated by the Response Adjudication Committee (RAC).

### Measurement of Eosinophils

Eosinophils in patients were measured at C1D15 (cycle 1, day 15) via a routine blood test, *see, e.g.,* LaGow B et al., eds. PDR Lab Advisor. A Comprehensive Point-of-Care Guide for Over 600 Lab Tests. 1st ed. Montvale, NJ: Thomson PDR, 2007; Pagana K, Pagana TJ eds. Mosby's Manual of Diagnostic and Laboratory Tests. 5th Ed. St. Louis, Missouri. 2014; https://www.ebmconsult.com/articles/lab-test-eosinophil-count and https://www.cancer.net/cancer-types/leukemia-eosinophilic/diagnosis.

The test results are demonstrated in Figures 1-4, which show that administration of Compound (I) significantly decreases number of absolute eosinophils in patients.

## Claims

1. A compound represented by the following structural formula: 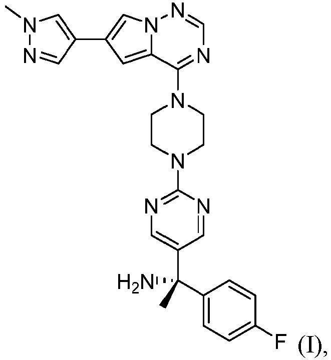 or a pharmaceutically acceptable salt thereof for use in treating an eosinophilic disorder.

2. The compound or a pharmaceutically acceptable salt thereof for use of claim 1, wherein the eosinophilic disorder is selected from hypereosinophilic syndrome, eosinophilia, eosinophilic enterogastritis, eosinophilic leukemia, eosinophilic granuloma and Kimura's disease.

3. The compound or a pharmaceutically acceptable salt thereof for use of claim 1, wherein the eosinophilic disorder is hypereosinophilic syndrome.

4. The compound or a pharmaceutically acceptable salt thereof for use of claim 1, wherein the eosinophilic disorder is eosinophilic leukemia.

5. The compound or a pharmaceutically acceptable salt thereof for use of claim 4, wherein the eosinophilic leukemia is chronic eosinophilic leukemia.

6. The compound or a pharmaceutically acceptable salt thereof for use of any one of claim 1-5, wherein the eosinophilic disorder is refractory to treatment with imatinib, sunitinib, and/or regorafenib.

7. The compound or a pharmaceutically acceptable salt thereof for use of any one of claims 1-6, wherein the subject treated for the eosinophilic disorder has a mutation in Exon 17 in KIT.

8. The compound or a pharmaceutically acceptable salt thereof for use of any one of claims 1-7, wherein the subject treated for the eosinophilic disorder has a D816 mutation in KIT in Exon 17.

9. The compound or a pharmaceutically acceptable salt thereof for use of claim 8, wherein the D816 mutation is D816V.

10. The compound or a pharmaceutically acceptable salt thereof for use of claim 8, wherein the D816 mutation is D816Y.

## Patentansprüche

1. Verbindung, dargestellt durch die folgende Strukturformel: 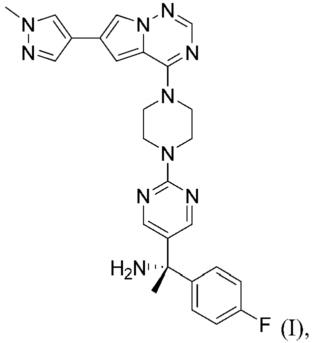 oder ein pharmazeutisch akzeptables Salz davon zur Verwendung bei der Behandlung einer eosinophilen Störung.

2. Verbindung oder pharmazeutisch akzeptables Salz davon zur Verwendung nach Anspruch 1, wobei die eosinophile Störung aus Hypereosinophilem Syndrom, Eosinophilie, eosinophiler Entergogastritis, eosinophiler Leukämie, eosinophilem Granulom und Morbus Kimura ausgewählt ist.

3. Verbindung oder pharmazeutisch akzeptables Salz davon zur Verwendung nach Anspruch 1, wobei die eosinophile Störung Hypereosinophiles Syndrom ist.

4. Verbindung oder pharmazeutisch akzeptables Salz davon zur Verwendung nach Anspruch 1, wobei die eosinophile Störung eosinophile Leukämie ist.

5. Verbindung oder pharmazeutisch akzeptables Salz davon zur Verwendung nach Anspruch 4, wobei die eosinophile Störung chronische eosinophile Leukämie ist.

6. Verbindung oder pharmazeutisch akzeptables Salz davon zur Verwendung nach einem der Ansprüche 1-5, wobei die eosinophile Störung refraktär gegenüber einer Behandlung mit Imatinib, Sunitinib und/oder Regorafenib ist.

7. Verbindung oder pharmazeutisch akzeptables Salz davon zur Verwendung nach einem der Ansprüche 1-6, wobei das Subjekt, das auf die eosinophile Störung behandelt wird, eine Mutation im KIT Exon 17 aufweist.

8. Verbindung oder pharmazeutisch akzeptables Salz davon zur Verwendung nach einem der Ansprüche 1-7, wobei das Subjekt, das auf die eosinophile Störung behandelt wird, eine D816-Mutation im KIT Exon 17 aufweist.

9. Verbindung oder pharmazeutisch akzeptables Salz davon zur Verwendung nach Anspruch 8, wobei die D816-Mutation D816V ist.

10. Verbindung oder pharmazeutisch akzeptables Salz davon zur Verwendung nach Anspruch 8, wobei die D816-Mutation D816Y ist.

## Revendications

1. Composé représenté par la formule structurale suivante : 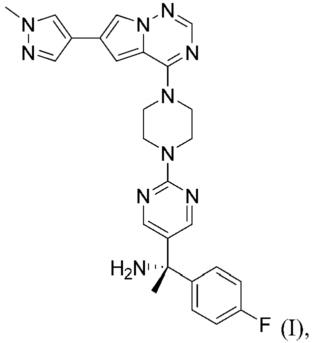 ou un sel pharmaceutiquement acceptable de celui-ci destiné à être utilisé dans le traitement d'un trouble éosinophilique.

2. Composé ou un sel pharmaceutiquement acceptable de celui-ci destiné à être utilisé selon la revendication 1, dans lequel le trouble éosinophilique est sélectionné parmi le syndrome hyperéosinophilique, l'éosinophilie, l'entérogastrite éosinophilique, la leucémie à éosinophiles, le granulome éosinophilique et la maladie de Kimura.

3. Composé ou un sel pharmaceutiquement acceptable de celui-ci destiné à être utilisé selon la revendication 1, dans lequel le trouble éosinophilique est le syndrome hyperéosinophilique.

4. Composé ou un sel pharmaceutiquement acceptable de celui-ci destiné à être utilisé selon la revendication 1, dans lequel le trouble éosinophilique est la leucémie à éosinophiles.

5. Composé ou un sel pharmaceutiquement acceptable de celui-ci destiné à être utilisé selon la revendication 4, dans lequel la leucémie à éosinophiles est la leucémie à éosinophiles chronique.

6. Composé ou un sel pharmaceutiquement acceptable de celui-ci destiné à être utilisé selon l'une quelconque des revendications 1 à 5, dans lequel le trouble éosinophilique est réfractaire à un traitement avec de l'imatinib, le sunitinib, et/ou le régorafénib.

7. Composé ou un sel pharmaceutiquement acceptable de celui-ci destiné à être utilisé selon l'une quelconque des revendications 1 à 6, dans lequel le sujet traité pour le trouble éosinophilique a une mutation dans Exon 17 dans KIT.

8. Composé ou un sel pharmaceutiquement acceptable de celui-ci destiné à être utilisé selon l'une quelconque des revendications 1 à 7, dans lequel le sujet traité pour le trouble éosinophilique a une mutation D816 dans KIT dans Exon 17.

9. Composé ou un sel pharmaceutiquement acceptable de celui-ci destiné à être utilisé selon la revendication 8, dans lequel la mutation D816 est D816V.

10. Composé ou un sel pharmaceutiquement acceptable de celui-ci destiné à être utilisé selon la revendication 8, dans lequel la mutation D816 est D816Y.
